# EUROPEAN PATENT SPECIFICATION

(11) **EP 3 593 904 B1**
(45) Date of publication and mention of the grant of the patent: **28.04.2021**
(21) Application number: 19183764.0
(22) Date of filing: 02.07.2019
(51) Int. Cl.: B01L 9/06, B01L 3/00, G01N 35/00, G06Q 10/08

(54) **METHOD AND SYSTEM FOR MANAGING THE STORAGE OF OBJECTS**
VERFAHREN UND SYSTEM ZUR VERWALTUNG DER AUFBEWAHRUNG VON OBJEKTEN
PROCÉDÉ ET SYSTÈME DE GESTION DU STOCKAGE D'OBJETS

(30) Priority: 11.07.2018 ES 201830692
(43) Date of publication of application: 15.01.2020
(73) Proprietor: Menarini Diagnósticos, S.A., 08918 Badalona (Barcelona) (ES)
(72) Inventor: FALCÓ PEGUEROLES, Pedro Félix, 08918 Badalona (Barcelona) (ES)
(74) Representative: Ungria López, Javier

(56) References cited:
- EP-A1- 2 080 553
- EP-A2- 2 755 034
- WO-A1-2010/116216
- WO-A1-2015/112791
- US-A1- 2016 328 588

## Description

### OBJECT OF THE INVENTION

The present invention relates to the technical field of the storage of objects and, more specifically, to the storage, identification and selection of objects, such as for example laboratory samples, with the assistance of certain visual indicators.

### BACKGROUND OF THE INVENTION

Currently, one of the most important points in the management of a laboratory is the correct management of the flows of liquid specimens (i.e. serum, plasma, blood, urine, etc.) in order to optimize work and performance.

Thus, laboratories are designed in such a way that higher volume tests are fully automated, usually in the form of chains wherein a sample is passed through several machines that use it if there are pending tests, or aliquot machines which generate several aliquots of the sample to be used in several machines.

In general, it can be said that there are valid and satisfactory solutions for the management of samples in higher volume processes of analytical laboratories, for example in documents US 2016/328588 A1 or WO 2010/116216 A1, but there are departments outside the main circuits wherein the current solutions are not valid for several reasons, such as:
- departments with small to medium volumes (50-400 samples/day) wherein the costs of valid solutions for large volumes are unaffordable;
- laboratories not integrated into the main work flows;
- laboratories wherein the tests to be carried out vary according to the results obtained, wherein more than performing a specific test, a request is received and the sample is characterized according to that request, which may require several tests in a sequential manner, depending on the previous results and combining several techniques. This implies temporary and localized storage of the sample;
- laboratories wherein there is a wide variability of technologies.

In these laboratories it is common to receive 50 to 400 samples a day that are stored in different sized racks, organized in different ways (usually one rack per day), wherein the samples must be manually ordered according to different criteria such as technique, sequential number, etc.

Among other problems, when a technique must be processed in one of these laboratories, a paper list is obtained from the Laboratory Information System (LIS) organized in different manners, according to which the samples should be searched for in the racks. As this search is manual, the process usually requires between several hours/person/day, which is an important fraction of the resources of the laboratory/department.

Therefore, in the state of the art a solution is needed for classifying and retrieving samples that is adapted to the needs of these laboratories and that is fast, efficient and cheap.

### DESCRIPTION OF THE INVENTION

In order to achieve the objectives and avoid the aforementioned drawbacks, the present invention describes, in a first aspect, a system for managing the storage of an object, wherein the system comprises: at least one linear support with multiple object receiving chambers arranged longitudinally, wherein position information of at least one of the chambers is previously recorded on a first label arranged on the linear support and readable with optical means; a reading base with optical reading means configured to obtain the position information of the at least one chamber and identification information of the object to be stored, wherein said identification information is previously recorded on a second label arranged on the object and readable with optical means; a receiving tray for linear supports coupled on the reading base, wherein the receiving tray comprises at least one guide rail, attachable to the linear support, which enables a movement of the linear support along the guide rail and wherein said movement results in an ordered succession of readings, with the optical means, of the first and second labels arranged on the linear support and the object located in the receiving chamber respectively; a control module in communication with the reading base, configured to receive the position and identification information obtained by the optical means, to determine a relative position of the object arranged in the receiving chamber of the linear support coupled in the guide rail, to store in a database said relative position associated with the identification information obtained from the object, and to send an activation signal to a certain visual indicator in response to a request for collection, by a user, of a previously stored object; and a plurality of visual indicators arranged in proximity to each of the receiving chambers of the linear support, configured to receive the activation signal from the control module and to be illuminated as a result of receiving said activation signal; characterized in that the receiving tray is a removable tray comprising first electrical contacts in correspondence with second electrical contacts arranged in the reading base in order to connect the tray to the base, and the tray further comprises holes in correspondence with pivots, arranged in the reading base, in order to couple the removable tray on the reading base in a specific position.

In one embodiment of the invention, the visual indicators are arranged in the linear support, wherein each of the visual indicators is associated, by proximity, to one of the receiving chambers of the linear support.

The present invention, in one of the embodiments thereof, further comprises a panel with lights attachable to the receiving tray, wherein the plurality of visual indicators are arranged in said panel with lights such that, in a coupled position of the panel with lights on the receiving tray, each of the visual indicators is associated, by proximity, with one of the receiving chambers for objects.

According to a particular embodiment, the present invention envisages dividing walls arranged between the rails of the receiving tray, wherein the visual indicators are arranged in said dividing walls such that each of the visual indicators is associated, by proximity, with one of the receiving chambers of the linear support completely inserted into the guide rail of the receiving tray.

Additionally, the present invention contemplates incorporating USB connection means, wherein the reading base comprises at least one USB female connector able to be connected to panel with lights.

One of the embodiments of the invention further comprises at least one sensor arranged in the guide rail, wherein said sensor is configured to detect the presence of a linear support in said guide rail and transmit detection information to the control module, wherein the control module is further configured, in response to the detection information, to send a reading command to the optical means of the reading base.

In one of the particular embodiments of the present invention, fastening means are additionally envisaged between the linear support and the receiving tray, wherein the fastening means are based on a male-female coupling comprising tabs in the base of the linear support, which are arranged in correspondence with slots in the guide rail of the receiving tray.

The optical reading means, according to one of the particular embodiments of the invention, are arranged perpendicularly to the direction of the guide rail of the receiving tray, such that the optical reading means emit a beam of light which intersects the linear supports in an entry point of the guide rail.

Additionally, in one of the embodiments of the invention, retention means are envisaged between the linear support and the receiving tray, wherein the linear support comprises, at one of the upper ends thereof, a clamp and the receiving tray comprises stops arranged on the upper portion of the wall opposite from the entry of the guide rail, such that the linear support completely inserted into the guide rail abuts with said wall and is temporarily retained by the mechanical engagement between the clamp and the stops.

A second aspect of the invention relates to a method for managing the storage of an object, wherein the method comprises the following steps:
a) recording position information of at least one object receiving chamber of a linear support on a first label arranged on said linear support and readable with optical means;
b) recording identification information of the object on a second label arranged on said object and readable with optical means;
c) coupling the linear support to a guide rail of a receiving tray, coupled in turn on a reading base comprising optical reading means, wherein the linear support comprises the object in the at least one object receiving chamber;
d) moving the linear support along the guide rail;
e) as a result of the movement of the linear support, identifying, with the optical means of the reading base, the first and second labels arranged on the linear support and the object respectively;
f) sending the position information of the chamber and the identification information of the object to be stored, recorded on the identified tags, to a control module;
g) determining, with the control module, a relative position of the object arranged in the receiving chamber of the linear support coupled in the guide rail;
h) storing, in a database of the control module, the relative position associated with the identification information of the object;
i) making a collection request, by a user interacting with the control module, based on the identification information of a previously stored object;
j) in response to the collection request, sending an activation instruction, from the control module, to a specific visual indicator, located in proximity to the receiving chamber of the linear support corresponding to the relative position information associated in the database with the identification information of the stored object; and
k) activating the certain visual indicator, in response to the activation instruction sent from the control module, to indicate to the user in which receiving chamber the object to be collected is located.

Optionally, in one of the embodiments of the present invention, the following additional steps are envisaged: detecting the presence of a linear support in the guide rail by means of a presence sensor arranged in said guide rail; transmitting detection information to the control module; in response to the detection information, sending a reading command from the control module to the optical means of the reading base; and emitting a beam of light from the optical means focused on the entry of the guide rail in order to perform the reading of the corresponding labels.

Additionally, in one of the embodiments of the invention it is envisaged that it completes a map of positions in the database according to the object identification information and the corresponding relative position information thereof, determined by the control module.

### BRIEF DESCRIPTION OF THE FIGURES

To complete the description of the invention, and for the purpose of helping to make the features thereof more readily understandable, according to a preferred exemplary embodiment thereof, a set of drawings is included where, by way of illustration and not limitation, the following figures have been represented:
- **Figure 1** represents a linear support with incorporated visual indicators, according to one of the embodiments of the invention.
- **Figure 2** shows a portable receiving tray, wherein the guide rails and male-female fastening means for the linear supports are shown. There are electrical contacts in the area in contact with the linear supports and the base of the receiving tray which transmit information between the base of Figure 4 and the linear supports.
- **Figure 3** shows a portable receiving tray with several linear supports placed therein.
- **Figure 4** shows a reading base according to one of the embodiments of the invention, configured to be able to be coupled to a portable receiving tray, and which contains a fixed barcode reader, reliefs for fitting said portable receiving tray, contacts connecting to said tray and connection to the central control unit.
- **Figure 5** shows the reading base with the portable receiving tray already coupled and several linear supports therein, one of which is partially inserted into one of the rails, such that it intersects the optical path of the barcode reader.
- **Figure 6** shows an embodiment of the invention wherein the reading base has guides for directly loading the linear supports and the LED indicators are in the structure of the reading base and not in the linear supports.
- **Figure 7** shows an embodiment of the invention wherein the visual indicators are arranged in a panel with lights, able to be coupled on the receiving tray.
- **Figure 8** shows an embodiment of the invention wherein the linear supports have no LEDs, but these LEDs are located in the receiving panel.

### DETAILED DESCRIPTION OF THE INVENTION

The present invention discloses a method and a system which facilitates the storage and subsequent search and retrieval of stored objects, such as for example laboratory sample tubes. It is based on the combination of a linear support device, such as a rack for example, which receives the objects and the identification of the relative position of said objects by reading identification barcodes and a limited and calibrated movement of said linear support with respect to a barcode reader. Once the objects are recorded in a central computer connected to the reader, they can be retrieved easily and quickly depending on any of the identifying characteristics that have been previously assigned to said central computer following simple visual indications, either through a screen or through a lighting system.

The limited and calibrated movement of the linear supports consists of the retention of the linear supports in rails which guide the linear supports in a linear movement of entry and exit on a tray. This limited movement is calibrated by an arrangement of barcodes on one side of the linear support which enables the locations able to receive objects to be identified. In this manner, the objects deposited in a linear support are sequentially identified and recorded as said linear support moves (according to the limited movement) along a rail perpendicular to the reading axis of a barcode reader. Once the identity and position of an object have been identified, the central control module stores both associated parameters in a position map.

One of the advantageous applications of the present invention relates to the storage of and search for liquid biological samples (serum, blood, urine, etc.) in tubes under analysis and study in research or diagnostic laboratories, but it is applicable to many other objects and scenarios.

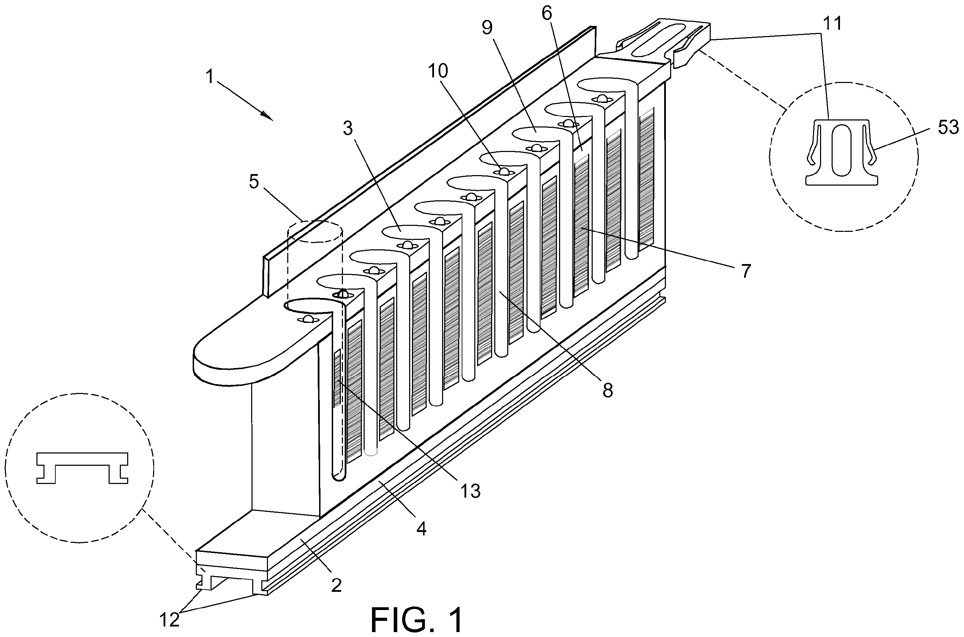

The linear support **1,** according to one of the embodiments shown in **Figure 1****,** is an elongated body **2** with a plurality of receiving chambers **3** for objects arranged perpendicular to a common base **4** and parallel to each other. The chambers are hollow, preferably identical bodies and are designed to receive in each of them a laboratory sample **5.** The space **6** defined between every two chambers is used to place a barcode **7** with position information relating to the adjacent chambers. The side wall of each of the chambers contains an opening **8.** The inner wall **9** of each of the chambers is used to place another barcode with information relating to the occupation of the chamber, wherein said code will be readable through the opening **8** in the side wall only if the chamber is not occupied by any object. If there is an object in the chamber, a sample tube for example, the very tube and the identification barcode **13** which is available therein prevent the reading of the inner code. The barcodes can be read by an optical reader oriented towards the side of the linear support wherein the barcodes are arranged.

The linear support can contain additional barcodes at the beginning or at the end thereof, which indicate features such as the number of tubes it can hold, the diameter thereof, a unique identification number of the support, etc. In one of the embodiments of the invention, the linear supports also contain a visual indicator **10** (for example, LED) in the upper area, next to each tube. These LEDs are connected to a small internal chip which further contains a unique identifier for each support and the chip is connected to one or several metallic contacts at the base of the support for the power and data transmission functions.

The linear support, according to one of the embodiments, is provided with a securing clamp **11** at one of the upper ends thereof to securely fasten it to a rail of a receiving tray or a transport tray. Additionally, in order to increase the firmness of the fastening of the linear support to a rail of a receiving tray or a transport tray, it can also have tabs **12** along the base of the linear support, configured to make a male-female set with the corresponding slots arranged in a rail of the receiving tray or transport tray.

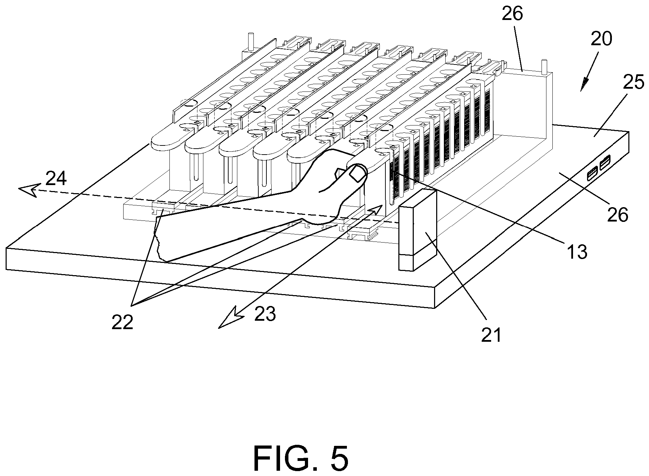

In **Figure 5****,** a reading module **20** is shown, which is formed by a reading base **25** and, forming a single body with the base, a tray **26** adapted to receive one or several linear supports **1,** identifiable by a unique barcode for each of them, and enabling the reading from the optical reader **21** of barcodes.

The tray has an arrangement of parallel rails **22** which are adapted to guide the linear supports in a movement **23** that is linear and perpendicular to the optical path of light **24** emitted by the optical reader. Thus, the linear supports, when inserted into each of said rails, intersect the beam of light **24** emitted by the optical reader and, in this manner, the optical reader **21** identifies the samples contained by the linear support in each of the chambers as said support moves linearly along the rail **22** of the tray and barcodes **13** are read in an ordered manner. Consequently, the corresponding electrical signals and control signals are generated which power the central control module.

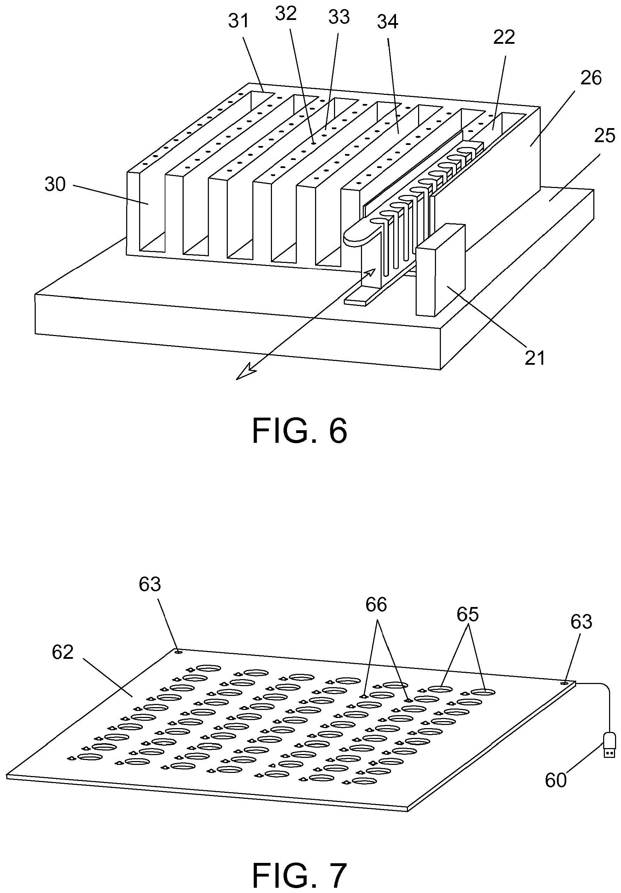

The rails of the tray can be made in several ways, in one of said embodiments which is shown in more detail in **Figure 6****,** dividing walls **34** are provided which delimit the rails, in addition to the guides of the base, which have an open entry end **30** to receive the linear supports and a width **31** designed in correspondence with that of the linear supports, such that the linear support inserted into the rail is retained on the transverse axis, but is kept free along the longitudinal axis of the support. The dividing walls have the main object of containing the visual indicators **32,** such as, for example, LED sample identifiers, in one of the embodiments of the invention.

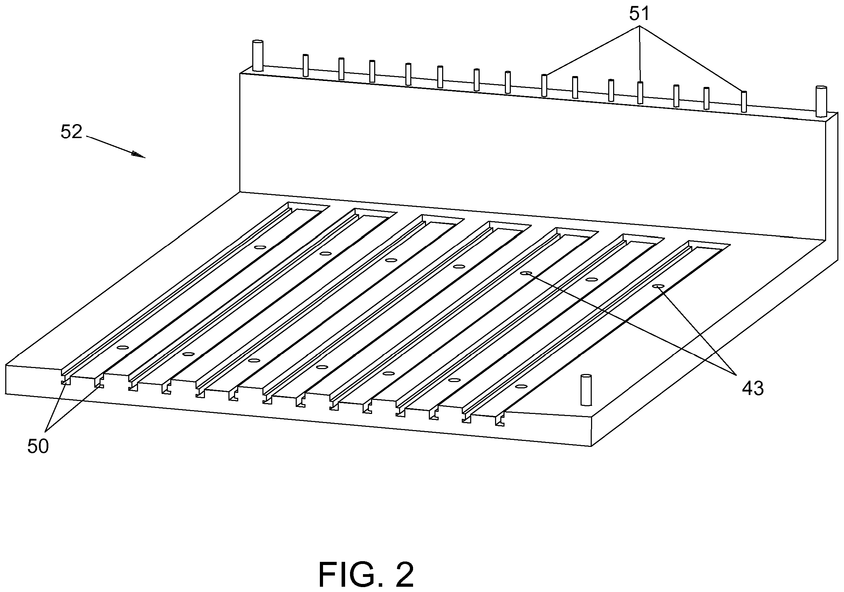

In an alternative embodiment, shown in **Figure 2****,** the rails of the tray are preferably arranged without dividing walls. Instead, although they are not incompatible features and can be combined in one same embodiment, the guide of the linear supports is made by arranging slots **50** in the base of each of the rails in correspondence with the tabs **12** arranged in the base of the linear supports, such that said linear supports can be joined to the tray by a male-female coupling. The male-female joint provides more firmness to the joint of the linear supports with the rail, but does not affect the longitudinal movement of the linear support in said rail.

Additionally, according to any of the previous guide configurations, stops **51** which hook with the securing clamp **11** of the linear support can be arranged in the upper portion of the wall, opposite from the open end of the rails. In this manner, the linear support, once it is fully inserted into the rail, is also retained longitudinally. The retention can be released by exerting a small pressure on the sides 53 of the clamp, necessary to remove the linear support from the rail or to perform reading operations.

At the base of the reading module **20** an optical barcode reader **21** is arranged, which emits a beam of light **24** perpendicular to the entry/exit direction **23** of the linear supports in the rails **22** which detects any barcode placed on a support which moves through any of the rails of the reading base. In one of the embodiments of the invention, the optical reader is located in a fixed position in one corner of the base and is oriented parallel to one of the sides thereof, the tray being quadrangular in shape, and at a distance from the closest side such that the beam of light it emits does not intersect the rails of the tray, but is as close as possible to the entry of said rails. The reader communicates with the central control module and sends the electrical and control signals corresponding to each reading to it.

The central control module comprises a central computer configured to receive the signals generated by the optical reader and associate the information contained in the barcodes with information previously loaded in the central computer. In the central control module, the position of each of the identified samples is recorded exactly, for example in a position matrix, given that the linear movement of the linear supports causes a reading of the barcodes of the samples in an ordered manner. Therefore, once the dimensions of the linear supports are known, as well as the number of available receiving chambers for objects, the central control module has an empty map of the possible positions to be occupied by the samples. As the linear supports move along the rails arranged in the receiving tray, the central module receives the readings made from the barcode reader in a sequential manner and fills the map with said readings. Additionally, the empty chambers are also identified by reading the barcode placed on the inner wall of the chambers. Finally, each of the positions of the position map of the central control module is associated with the identification information recorded in the corresponding barcodes, such as for example type of sample, date, user, analysis, test to be performed or others.

A graphic interface is arranged in the central control module to enable a user to interact with the system and define the search parameters, dimensions of the supports, types of samples or any other identifying detail thereof.

Once the map of the positions of the samples identified in the central control module has been completed, it is possible to locate any of the samples by entering any of the identifying data that has been stored associated with said sample. The user enters the chosen parameter through the graphical interface of the central module and this module retrieves from the database thereof the position occupied by said sample in the position map. Once the central module has determined the position occupied by the sample searched for, it proceeds to send an activation signal to the visual indicator associated with said position. In this manner, the user receives a clear visual indication of which one is the sample that must be collected easily and quickly.

The visual indicators can be implemented preferably in the linear supports, as explained above and can be seen in Figure 1 or in the tray of the reading module, as seen in Figure 6. For example, according to one of the embodiments shown in Figure 6, a visual indicator **32** is arranged, as a light-emitting diode (LED), in the upper profile **33** of the wall **34** that separates adjacent rails of the tray next to each one of the possible positions of the samples, such that a flash or a light of a certain colour is emitted only in the indicator located next to the sample to be collected. Optionally, lighting devices of several colours can be included in one same position, in order to enable multiple searches. The LED lights are connected to the central control module and are responsive to the electrical control signals emitted from it.

Additionally, it can be verified that the object removed from the linear support (following the indications of the central control module) is indeed the indicated object. To do so, in one embodiment of the invention an external reading of the identifier of the object removed by the operator is envisaged (for example, the reading of the barcode by means of an external barcode reader every time an object is manually selected), or, according to another embodiment, by a complete reading of all the positions of the linear support, wherein it is verified that the positions with identified objects are unoccupied (which means that the operator has selected the correct object).

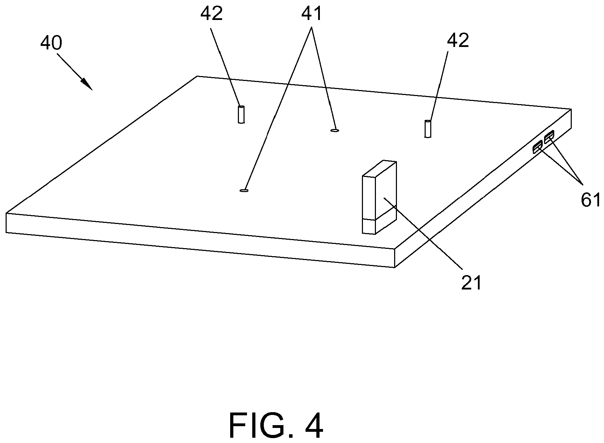

In one embodiment of the invention, the reading module has two parts which can be coupled together, on one side the reading base **40,** which is represented isolated in the **Figure 4****,** and on the other a tray **52,** such as the one shown in **Figure 2****,** with the particularity that both parts are removable. To do so, the reading base comprises electrical contacts **41** and fastening means, for example pivots **42** which fit into holes made in the lower part of the removable tray in order to couple said removable tray in a specific position on the reading base. The coupled position of both parts can be seen in **Figure 5****.**

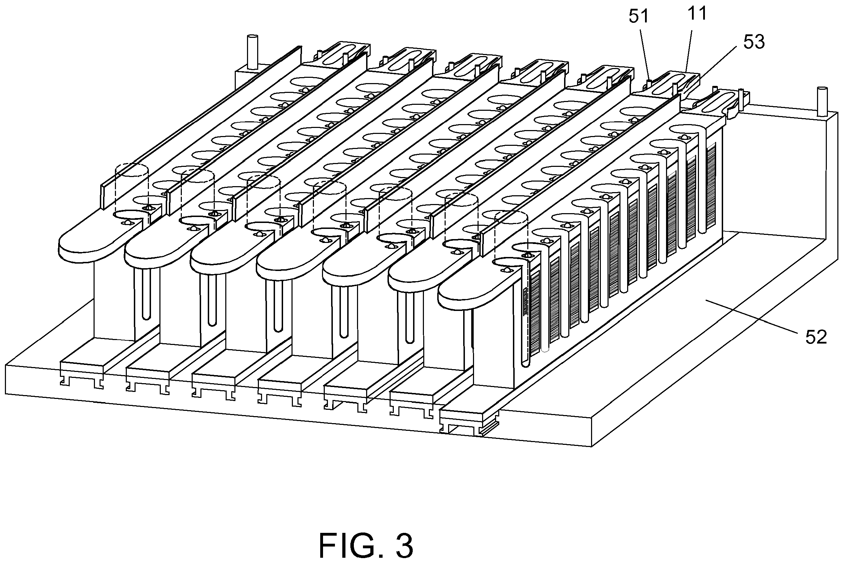

This removable tray can comprise any of the rail arrangements described above, such as dividing walls and/or slots in the base able to be coupled by a male-female coupling to the linear supports (as in the case of the tray **52** shown in **Figure 3****),** for the reception and storage of linear supports while these supports are not required for any test.

The removable trays are identified manually by the operator in order to relate the identified objects to the transport/storage unit, for example with a label intelligible to the user. They can also be identified by barcode; by radiofrequency (RFID) techniques, adhering RFID tags on said trays and the corresponding RFID readers in the reading base; or by any other method of identification for use, such as an internal identification chip of the removable tray and electrical contacts for the reading bases through which the power and data of the bases is transmitted. The removable trays can store the tubes in the refrigerator for as long as necessary and be attached to each other in order to form larger structures, both for transport and storage.

Additionally, in one of the embodiments of the invention, the trays, whether removable trays or trays permanently joined to the reading base, have sensors **43** which detect if there are linear supports loaded. To read the tubes contained in the linear supports, determine the position thereof and for these to be identified, the linear support moves on one of the rails of the tray, such that these sensors **43** detect the movement of the linear support and indicate to the optical reader where the beam of light should be focused. Additionally, the sensor transmits the identification of the linear support to the reading base, data and the power of said linear support.

The removable trays can incorporate visual indicators for indicating the position of a desired object (sample). In this case, the activation of the luminous indicators, preferably LEDs, can be carried out through a wireless communication between the removable tray and the central control module, as an alternative to the option of physically connecting the removable tray and the central control module by means of the corresponding physical connectors, wherein the electrical path of the removable tray to the central control module, according to one of the embodiments of the invention, consists of electrical contacts **41** between the removable tray and the reading base, combined in turn with a pair of female USB connectors **61** arranged on one side of the base of the reading module.

One of the advantages of the embodiments of the invention based on removable elements is that it is not necessary to remove the linear supports from a receiving or transport tray in order to place them in the reading module, but rather, the removable trays themselves can be identified and form storage superstructures to store and subsequently transport the linear supports and, with all the linear supports, they can be coupled directly on the reading base. For the recording of the linear supports, it will be necessary to linearly move each of the linear supports (without removing them completely from the tray) with the samples, so that the beam of light emitted from the barcode reader intersects and detects all the barcodes.

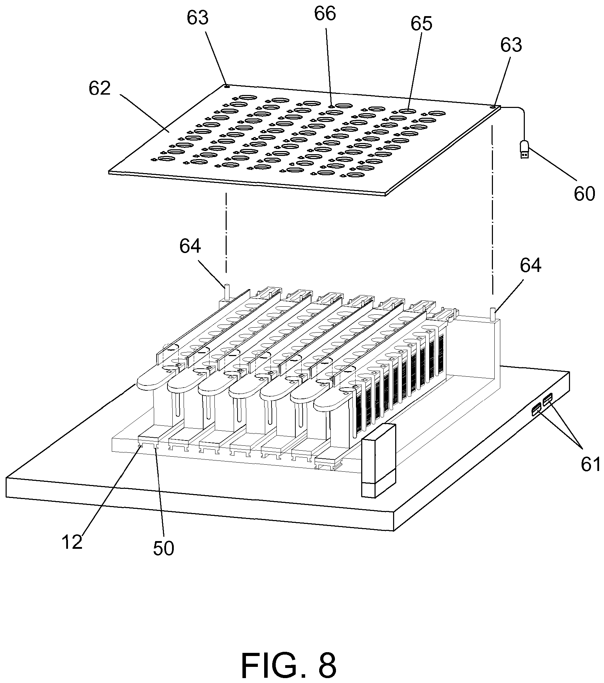

The visual indicators, according to one embodiment of the invention, are located in external elements compatible with the reading module, such as, for example, the panels with lights **62** shown in **Figure 8****.** In it, a modular configuration of the present invention can be seen in three parts, meaning, the reading base, the removable tray coupled onto it and a panel with lights which can be coupled on the assembly made up of the reading base and the removable tray.

Coupling means are provided between the panel with lights and the tray. The panel with lights includes perforations **63** in the back corners in correspondence with pivots **64** arranged on the rear wall of the tray. When the holes are inserted into the pivots, the position for coupling between the panel and the tray causes the total convergence of the receiving chambers for the linear supports and an arrangement of holes **65** of the panel with lights. This convergence enables sample tubes to be inserted into and removed from the linear supports in the coupling position of the panel and the tray. The panel with lights has an arrangement of visual indicators, preferably LED lights **66,** located next to the holes, such that in the coupled position, each of the visual indicators is associated with one of the positions of the linear supports.

The communication between the panel and the central control module can be based on RFID communications or electrical contacts between the panel and the tray, such as a USB connector **60** that makes use of any of the female USB connectors **61** which are arranged in the base of the reading module in some embodiments of the invention, such that the central control module can send the electrical and control signals necessary for the activation of the corresponding lights.

Part of the infrastructure that can be used with the embodiments described in this document is already available, such as: general purpose computers, computer programming tools and techniques, digital storage means, and communication networks. A control module is understood to include a computer and/or a processor, such as a microprocessor, microcontroller, logic circuitry, or similar. The processor may include a special purpose processing device, such as an ASIC, PAL, PLA, PLD, programmable field gate array, or another customized or programmable device. The computer may also include a computer readable storage device, such as non-volatile memory, static RAM, dynamic RAM, ROM, CD-ROM, disk, tape, magnetic, optical, flash memory, or other computer-readable storage means.

The present invention should not be limited by the embodiment herein described. Other configurations may be carried out by those skilled in the art based on the present description. Accordingly, the scope of the invention is defined by the following claims.

## Claims

1. A system for managing the storage of an object comprising:
- at least one linear support (1) with multiple object receiving chambers (3) arranged longitudinally, wherein position information of at least one of the chambers is previously recorded on a first label (7) arranged on the linear support and readable with optical means;
- a reading base (25) with optical reading means (21) configured to obtain the position information of the at least one chamber and identification information of the object to be stored, wherein said identification information is previously recorded on a second label (13) arranged on the object and readable with optical means;
- a receiving tray (26) for linear supports coupled on the reading base, wherein the receiving tray comprises at least one guide rail (22), attachable to the linear support, which enables a movement (23) of the linear support along the guide rail and wherein said movement results in an ordered succession of readings, by the optical means, of the first and second labels arranged on the linear support and the object located in the receiving chamber respectively;
- a control module in communication with the reading base, configured to receive the position and identification information obtained by the optical means, to determine a relative position of the object arranged in the receiving chamber of the linear support coupled in the guide rail, to store in a database said relative position associated with the identification information obtained from the object, and to send an activation signal to a certain visual indicator in response to a request for collection, by a user, of a previously stored object; and
- a plurality of visual indicators (10, 32, 66) arranged in proximity to each of the receiving chambers of the linear support, configured to receive the activation signal from the control module and to be illuminated as a result of receiving said activation signal;
**characterized in that** the receiving tray is a removable tray (52) comprising first electrical contacts (41) in correspondence with second electrical contacts arranged in the reading base in order to connect the tray to the base, and the tray further comprises holes in correspondence with pivots (42), arranged in the reading base, in order to couple the removable tray on the reading base in a specific position.

2. The system according to claim 1, wherein the visual indicators are arranged in the linear support, wherein each of the visual indicators is associated by proximity, to one of the receiving chambers of the linear support.

3. The system according to claim 1, which further comprises a panel with lights (62), attachable to the receiving tray, wherein the plurality of visual indicators (66) are arranged in said panel with lights such that, in a coupled position of the panel with lights on the receiving tray, each of the visual indicators is associated, by proximity, with one of the receiving chambers for objects.

4. The system according to claim 1, which further comprises dividing walls (34) arranged between the rails of the receiving tray, wherein the visual indicators are arranged in said dividing walls such that each of the visual indicators is associated, by proximity, with one of the receiving chambers of the linear support completely inserted into the guide rail of the receiving tray.

5. The system according to claim 3, which further comprises USB connection means, wherein the reading base comprises at least one female USB connector (61) able to be connected to the panel with lights.

6. The system according to any of the preceding claims, which further comprises at least one sensor (43) arranged in the guide rail, wherein said sensor is configured to detect the presence of a linear support in said guide rail and transmit detection information to the control module, wherein the control module is further configured, in response to the detection information, to send a reading command to the optical means of the reading base.

7. The system according to any of the preceding claims, which further comprises fastening means between the linear support and the receiving tray, wherein the fastening means are based on a male-female coupling comprising tabs (12) in the base of the linear support, which are arranged in correspondence with slots (50) in the guide rail of the receiving tray.

8. The system according to any of the preceding claims, wherein the optical reading means are arranged perpendicular to the direction of the guide rail of the receiving tray, such that the optical reading means emit a beam of light (24) which intersects the linear supports in an entry point of the guide rail.

9. The system according to any of the preceding claims, which further comprises retention means between the linear support and the receiving tray, wherein the linear support comprises, at one of the upper ends thereof, a clamp (11) and the receiving tray comprises stops (51) arranged on the upper portion of the wall opposite from the entry of the guide rail, such that the linear support completely inserted into the guide rail abuts with said wall and is temporarily retained by the mechanical engagement between the clamp and the stops.

10. A method for managing the storage of an object with the system according to any of the preceding claims, **characterized in that** it comprises the following steps:
a) recording position information of at least one object receiving chamber (3) of a linear support (1) on a first label (7) arranged on said linear support and readable with optical means;
b) recording identification information of the object (5) on a second label (13) arranged on said object and readable with optical means;
c) coupling the linear support to a guide rail (22) of a receiving tray (26), coupled in turn on a reading base (25) comprising optical reading means (21), wherein the linear support comprises the object in the at least one object receiving chamber;
d) moving (23) the linear support along the guide rail;
e) as a result of the movement of the linear support, identifying, with the optical means of the reading base, the first and second labels arranged on the linear support and the object respectively;
f) sending the position information of the chamber and the identification information of the object to be stored, recorded on the identified tags, to a control module;
g) determining, with the control module, a relative position of the object arranged in the receiving chamber of the linear support coupled in the guide rail;
h) storing, in a database of the control module, the relative position associated with the identification information of the object;
i) making a collection request, by a user interacting with the control module, based on the identification information of a previously stored object;
j) in response to the collection request, sending an activation instruction, from the control module, to a specific visual indicator (10), located in proximity to the receiving chamber of the linear support corresponding to the relative position information associated in the database with the identification information of the stored object; and
k) activating the certain visual indicator, in response to the activation instruction sent from the control module, to indicate to the user in which receiving chamber the object to be collected is located.

11. The method according to claim 10, which further comprises:
- detecting the presence of a linear support in the guide rail by means of a presence sensor arranged in said guide rail;
- transmitting detection information to the control module;
- in response to the detection information, sending a reading command from the control module to the optical means of the reading base; and
- emitting a beam of light (24) from the optical means focused on the entry of the guide rail in order to perform the reading of the corresponding labels.

12. The method according to any one of claims 10-11, which further comprises completing a map of positions in the database according to the object identification information and the corresponding relative position information thereof, determined by the control module.

## Patentansprüche

1. System zur Verwaltung der Lagerung eines Objekts, mit:
- mindestens einer geraden Halterung (1) mit mehreren objektaufnehmenden Kammern (3), die in Längsrichtung angeordnet sind, wobei Positionsinformation mindestens einer der Kammern zuvor auf einem ersten Etikett (7), das auf der geraden Halterung angeordnet und mit einer optischen Einrichtung lesbar ist, aufgezeichnet wird;
- einer Lesebasiseinheit (25) mit einer optischen Leseeinrichtung (21), die ausgebildet ist, die Positionsinformation der mindestens einen Kammer und Identifizierungsinformation des zu lagernden Objekts zu erhalten, wobei die Identifizierungsinformation zuvor auf einem zweiten Etikett (13), das auf dem Objekt angeordnet und mit der optischen Einrichtung lesbar ist, aufgezeichnet wird;
- einem Aufnahmefach (26) für gerade Halterungen, das auf der Lesebasiseinheit angebracht ist, wobei das Aufnahmefach mindestens eine Führungsschiene (22), die an der geraden Halterung anbringbar ist, aufweist, die eine Bewegung (23) der geraden Halterung entlang der Führungsschiene ermöglicht, und wobei die Bewegung zu einer geordneten Abfolge von durch die optische Einrichtung ausgeführten Lesevorgängen für das erste und das zweite Etikett führt, die entsprechend auf der geraden Halterung und dem Objekt, das in der aufnehmenden Kammer angeordnet ist, angeordnet sind;
- einem Steuerungsmodul, das mit der Lesebasiseinheit in Verbindung steht und ausgebildet ist, die Positionsinformation und die Identifizierungsinformation, die mittels der optischen Einheit erhalten wird, zu empfangen, eine relative Position des in der aufnehmenden Kammer der geraden Halterung, die in der Führungsschiene angekoppelt ist, zu ermitteln, die relative Position, die der von dem Objekt erhaltenen Identifizierungsinformation zugeordnet ist, in einer Datenbank zu speichern, und ein Aktivierungssignal an einen gewissen visuellen Indikator in Reaktion auf eine Anforderung zur Einsammlung eines zuvor gelagerten Objekts, die von einem Benutzer stammt, zu senden; und
- mehreren visuellen Indikatoren (10, 32, 66), die in der Nähe entsprechend jeder der aufnehmenden Kammern der geraden Halterung angeordnet und ausgebildet sind, das Aktivierungssignal aus dem Steuerungsmodul zu empfangen und als Ergebnis des Empfangs des Aktivierungssignals aufzuleuchten;
**dadurch gekennzeichnet, dass**
das Aufnahmefach (42) ein bewegliches Fach (52) mit ersten elektrischen Kontakten (41) ist, die zweiten elektrischen Kontakten entsprechen, die in der Lesebasiseinheit angeordnet sind, um das Fach mit der Basiseinheit zu verbinden, und wobei das Fach ferner Bohrungen, die Drehpunkten (42) entsprechen, die in der Lesebasiseinheit angeordnet sind, aufweist, um das bewegliche Fach auf der Lesebasiseinheit in einer speziellen Position anzukoppeln.

2. System nach Anspruch 1, wobei die visuellen Indikatoren in der geraden Halterung angeordnet sind, wobei jeder der visuellen Indikatoren durch seine entsprechende Nähe einer der aufnehmenden Kammern der geraden Halterung zugeordnet sind.

3. System nach Anspruch 1, das ferner eine Tafel mit Leuchten (62) aufweist, die an dem Aufnahmefach anbringbar ist, wobei die mehreren visuellen Indikatoren (66) in der Tafel mit Leuchten derart angeordnet sind, dass in einer an das Aufnahmefach angekoppelten Position der Tafel mit Leuchten jeder der visuellen Indikatoren durch seine Nähe einer der objektaufnehmenden Kammern zugeordnet ist.

4. System nach Anspruch 1, das ferner Trennwände (34) aufweist, die zwischen den Schienen des Aufnahmefachs angeordnet sind, wobei die visuellen Indikatoren in den Trennwänden derart angeordnet sind, dass jeder der visuellen Indikatoren durch seine Nähe einer der aufnehmenden Kammern der geraden Halterung zugeordnet ist, die vollständig in die Führungsschiene des Aufnahmefachs eingeführt ist.

5. System nach Anspruch 3, das ferner USB-Verbindungseinrichtungen aufweist, wobei die Lesebasiseinheit mindestens einen USB-Buchsenstecker (61) aufweist, der mit der Tafel mit Leuchten verbindbar ist.

6. System nach einem der vorhergehenden Ansprüche, das ferner mindestens einen Sensor (43) aufweist, der in der Führungsschiene angeordnet ist, wobei der Sensor ausgebildet ist, die Anwesenheit einer geraden Halterung in der Führungsschiene zu erkennen und Erkennungsinformation an das Steuerungsmodul zu senden, wobei das Steuerungsmodul ferner ausgebildet ist, in Reaktion auf die Erkennungsinformation einen Lesebefehl an die optische Einrichtung der Lesebasiseinheit zu senden.

7. System nach einem der vorhergehenden Ansprüche, das ferner eine Befestigungseinrichtung zwischen der geraden Halterung und dem Aufnahmefach aufweist, wobei die Befestigungseinrichtung auf einer Stift-Buchsen-Kopplung beruht, mit Laschen (12) im Grundgestell der geraden Halterung, die entsprechend zu Einschnitten (50) in der Führungsschiene des Aufnahmefachs angeordnet sind.

8. System nach einem der vorhergehenden Ansprüche, wobei die optische Leseeinrichtung senkrecht zur Richtung der Führungsschiene des Aufnahmefachs angeordnet ist derart, dass die optische Leseeinrichtung einen Lichtstrahl (24) aussendet, der die geraden Halterungen an einem Eintrittspunkt der Führungsschiene schneidet.

9. System nach einem der vorhergehenden Ansprüche, das ferner eine Rückhalteeinrichtung zwischen der geraden Halterung und dem Aufnahmefach aufweist, wobei die gerade Halterung an einem ihrer oberen Enden eine Klemme (11) aufweist und das Aufnahmefach Anschläge (51) aufweist, die auf dem oberen Bereich der Wand gegenüberliegend zu dem Eingang der Führungsschiene angeordnet derart, dass die gerade Halterung, wenn sie vollständig in die Führungsschiene eingeführt ist, an der Wand anliegt und zeitweilig durch den mechanischen Kontakt zwischen der Klemme und den Anschlägen gehalten wird.

10. Verfahren zur Verwaltung der Lagerung eines Objekts mit einem System nach einem der vorhergehenden Ansprüche,
**dadurch gekennzeichnet, dass** das Verfahren die folgenden Schritte umfasst:
a) Aufzeichnen von Positionsinformation mindestens einer ein Objekt aufnehmenden Kammer (3) einer geraden Halterung (1) auf einem ersten Etikett (7), das auf der geraden Halterung angeordnet und mit einer optischen Einrichtung lesbar ist;
b) Aufzeichnen einer Identifizierungsinformation des Objekts (5) auf einem zweiten Etikett (13), das auf dem Objekt angeordnet und mit einer optischen Einrichtung lesbar ist;
c) Ankoppeln der geraden Halterung an eine Führungsschiene (22) eines Aufnahmefachs (26), das wiederum an eine Lesebasiseinheit (25) gekoppelt ist, die eine optische Leseeinrichtung (21) aufweist, wobei die gerade Halterung das Objekt in der mindestens einen objektaufnehmenden Kammer aufweist;
d) Bewegen (23) der geraden Halterung entlang der Führungsschiene;
e) als Folge der Bewegung der geraden Halterung, Erkennen des ersten und des zweiten Etiketts, die entsprechend an der geraden Halterung und an dem Objekt angeordnet sind, mittels der optischen Einrichtung der Lesebasiseinheit;
f) Senden der Positionsinformation der Kammer und der Identifizierungsinformation des zu lagernden Objekts, die auf den erkannten Etiketten aufgezeichnet sind, an ein Steuerungsmodul;
g) Ermitteln einer relativen Position des Objekts, das in der Aufnahmekammer der geraden Halterung angeordnet ist, die mit der Führungsschiene gekoppelt ist;
h) Speichern der relativen Position, die der Identifizierungsinformation des Objekts zugeordnet ist, in einer Datenbank des Steuerungsmoduls;
i) Erstellen einer Einsammelanforderung durch einen Benutzer, der mit dem Steuerungsmodul interagiert, auf der Grundlage der Identifizierungsinformation eines zuvor gelagerten Objekts;
j) in Reaktion auf die Einsammelanforderung, Senden eines Aktivierungsbefehls von dem Steuerungsmodul an einen speziellen visuellen Indikator (10), der in der Nähe der Aufnahmekammer der geraden Halterung entsprechend zu der relativen Positionsinformation, die in der Datenbank der Identifizierungsinformation des gelagerten Objekts zugeordnet ist, angeordnet ist; und
k) Aktivieren des gewissen visuellen Indikators in Reaktion darauf, dass der Aktivierungsbefehl von dem Steuerungsmodell gesendet wird, um für den Benutzer anzuzeigen, in welcher Aufnahmekammer das einzusammelnde Objekt angeordnet ist.

11. Verfahren nach Anspruch 10, das ferner umfasst:
- Erkennen der Anwesenheit einer geraden Halterung in der Führungsschiene mittels eines Präsenzsensors, der in der Führungsschiene angeordnet ist;
- Senden einer Erkennungsinformation an das Steuerungsmodul;
- in Reaktion auf die Erkennungsinformation, Senden eines Lesebefehls von dem Steuerungsmodul an die optische Einrichtung der Lesebasiseinheit; und
- Aussenden eines Lichtstrahls (24) aus der optischen Einrichtung, der auf den Eingang der Führungsschiene fokussiert ist, um das Lesen der entsprechenden Etiketten auszuführen.

12. Verfahren nach einem der Ansprüche 10-11, das ferner umfasst: Vervollständigen einer Karte von Positionen in der Datenbank gemäß der Objektidentifizierungsinformation und der entsprechenden Relativpositionsinformation, die von dem Steuerungsmodul ermittelt werden.

## Revendications

1. Système pour gérer le stockage d'un objet comprenant :
- au moins un support linéaire (1) avec plusieurs chambres de réception d'objet (3) agencées de manière longitudinale, dans lequel l'information de position d'au moins l'une des chambres est préalablement enregistrée sur une première étiquette (7) disposée sur le support linéaire et lisible avec des moyens optiques ;
- une base de lecture (25) avec des moyens de lecture optiques (21) configurés pour obtenir l'information de position de la au moins une chambre et l'information d'identification de l'objet à stocker, dans lequel ladite information d'identification est préalablement enregistrée sur une second étiquette (13) disposée sur l'objet et lisible avec des moyens optiques ;
- un plateau de réception (26) pour supports linéaires, couplé sur la base de lecture, dans lequel le plateau de réception comprend au moins un rail de guidage (22) pouvant être fixé au support linéaire, qui permet un mouvement (23) du support linéaire le long du rail de guidage et dans lequel ledit mouvement se traduit par une succession ordonnée de lectures, par les moyens optiques, des première et seconde étiquettes disposées sur le support linéaire et l'objet positionné dans la chambre de réception respectivement ;
- un module de commande en communication avec la base de lecture, configuré pour recevoir l'information de position et d'identification obtenue par les moyens optiques, afin de déterminer une position relative de l'objet agencé dans la chambre de réception du support linéaire couplé dans le rail de guidage, afin de stocker, dans une base de données, ladite position relative associée à l'information d'identification obtenue à partir de l'objet, et pour envoyer un signal d'activation à un certain indicateur visuel en réponse à une demande de collecte, par un utilisateur, d'in objet préalablement stocké ; et
- une pluralité d'indicateurs visuels (10, 32, 66) agencés à proximité de chacune des chambres de réception du support linéaire, configurés pour recevoir le signal d'activation du module de commande et être éclairés suite à la réception dudit signal d'activation ;
**caractérisé en ce que** le plateau de réception est un plateau amovible (52) comprenant des premiers contacts électriques (41) en correspondance avec des seconds contacts électriques agencés dans la base de lecture afin de raccorder le plateau à la base, et le plateau comprend en outre des trous en correspondance avec des pivots (42), agencés dans la base de lecture, afin de coupler le plateau amovible sur la base de lecture dans une position spécifique.

2. Système selon la revendication 1, dans lequel les indicateurs visuels sont agencés dans le support linéaire, dans lequel chacun des indicateurs visuels est associé par proximité, à l'une des chambres de réception du support linéaire.

3. Système selon la revendication 1, comprenant en outre un panneau avec des lumières (62), pouvant être fixé au plateau de réception, dans lequel la pluralité d'indicateurs visuels (66) sont agencés dans ledit panneau avec des lumières de sorte que, dans une position couplée du panneau avec les lumières sur le plateau de réception, chacun des indicateurs visuels est associé, par proximité, à l'une des chambres de réception pour objets.

4. Système selon la revendication 1, comprenant en outre des parois de division (34) agencées entre les rails du plateau de réception, dans lequel les indicateurs visuels sont agencés dans lesdites parois de division de sorte que chacun des indicateurs visuels est associé, par proximité, à l'une des chambres de réception du support linéaire complètement insérée dans le rail de guidage du plateau de réception.

5. Système selon la revendication 3, comprenant en outre des moyens de raccordement USB, dans lequel la base de lecture comprend au moins un connecteur USB femelle (61) pouvant être raccordé au panneau avec les lumières.

6. Système selon l'une quelconque des revendications précédentes, comprenant en outre au moins un capteur (43) agencé dans le rail de guidage, dans lequel ledit capteur est configuré pour détecter la présence d'un support linéaire dans ledit rail linéaire et transmettre l'information de détection au module de commande, dans lequel le module de commande est en outre configuré, en réponse à l'information de détection, pour envoyer une commande de lecture aux moyens optiques de la base de lecture.

7. Système selon l'une quelconque des revendications précédentes, comprenant en outre des moyens de fixation entre le support linéaire et le plateau de réception, dans lequel les moyens de fixation sont basés sur un couplage mâle - femelle comprenant des languettes (12) dans la base du support linéaire, qui sont agencées en correspondance avec des fentes (50) dans le rail de guidage du plateau de réception.

8. Système selon l'une quelconque des revendications précédentes, dans lequel les moyens de lecture optiques sont agencés perpendiculairement à la direction du rail de guidage du plateau de réception, de sorte que les moyens de lecture optiques émettent un faisceau de lumière (24) qui coupe les supports linéaires dans un point d'entrée du rail de guidage.

9. Système selon l'une quelconque des revendications précédentes, comprenant en outre des moyens de retenue entre le support linéaire et le plateau de réception, dans lequel le support linéaire comprend, au niveau de l'une de ses extrémités supérieures, un dispositif de serrage (11) et le plateau de réception comprend des butées (51) agencées sur la partie supérieure de la paroi opposée à l'entrée du rail de guidage, de sorte que le support linéaire complètement inséré dans le rail de guidage vient en butée avec ladite paroi et est temporairement retenu par la mise en prise mécanique entre le dispositif de serrage et les butées.

10. Procédé pour gérer le stockage d'un objet avec le système selon l'une quelconque des revendications précédentes, **caractérisé en ce qu'**il comprend les étapes suivantes consistant à :
a) enregistrer l'information de position d'au moins une chambre de réception d'objet (3) d'un support linéaire (1) sur une première étiquette (7) disposée sur ledit support linéaire et lisible par des moyens optiques ;
b) enregistrer l'information d'identification de l'objet (5) sur une seconde étiquette (13) disposée sur ledit objet et lisible avec les moyens optiques ;
c) coupler le support linéaire à un rail de guidage (22) d'un plateau de réception (26), couplé à son tour sur une base de lecture (25) comprenant des moyens de lecture optiques (21), dans lequel le support linéaire comprend l'objet dans la au moins une chambre de réception d'objet ;
d) déplacer (23) le support linéaire le long du rail de guidage ;
e) suite au mouvement du support linéaire, identifier, avec les moyens optiques de la base de lecture, les première et seconde étiquettes disposées sur le support linéaire et l'objet respectivement ;
f) envoyer l'information de position de la chambre et l'information d'identification de l'objet à stocker, enregistrées sur les étiquettes identifiées, à un module de commande ;
g) déterminer, avec le module de commande, une position relative de l'objet agencé dans la chambre de réception du support linéaire couplé dans le rail de guidage ;
h) stocker, dans une base de données du module de commande, la position relative associée avec l'information d'identification de l'objet ;
i) réaliser une demande de collecte, par un utilisateur qui interagit avec le module de commande, sur la base de l'information d'identification d'un objet préalablement stocké ;
j) en réponse à la demande de collecte, envoyer une instruction d'activation, du module de commande à un indicateur visuel (10) spécifique, positionné à proximité de la chambre de réception du support linéaire correspondant à l'information de position relative associée dans la base de données avec l'information d'identification de l'objet stocké ; et
k) activer le certain indicateur visuel, en réponse à l'instruction d'activation envoyée par le module de commande, pour indiquer à l'utilisateur dans quelle chambre de réception, se trouve l'objet à collecter.

11. Procédé selon la revendication 10, comprenant en outre l'étape consistant à :
- détecter la présence d'un support linéaire dans le rail de guidage au moyen d'un capteur de présence agencé dans ledit rail de guidage ;
- transmettre l'information de détection au module de commande ;
- en réponse à l'information de détection, envoyer une commande de lecture du module de commande aux moyens optiques de la base de lecture ; et
- émettre un faisceau de lumière (24) des moyens optiques concentré sur l'entrée du rail de guidage afin de réaliser la lecture des étiquettes correspondantes.

12. Procédé selon l'une quelconque des revendications 10 à 11, comprenant en outre l'étape consistant à compléter une carte de positions dans la base de données selon l'information d'identification d'objet et son information de position relative correspondante, déterminées par le module de commande.
